# FASCICULE DE BREVET EUROPEEN

(11) **EP 0 641 560 B1**
(45) Date de publication et mention de la délivrance du brevet: **15.05.1996**
(21) Numéro de dépôt: 94401912.4
(22) Date de dépôt: 29.08.1994
(51) Int. Cl.: A61K 9/20, A61K 9/00

(54) **Comprimé vétérinaire plus spécialement destiné aux chats**
Tierärztliche Tablette besonders für Katzen bestimmt
Veterinary tablet especially meant for cats

(30) Priorité: 01.09.1993 FR 9310422
(43) Date de publication de la demande: 08.03.1995
(73) Titulaire: SO.GE.VAL S.A., Laval Mayenne (FR)
(72) Inventeur: Daoudal, José, 53000 Laval (FR)
(74) Mandataire: Cabinet Pierre HERRBURGER

(56) Documents cités:
- EP-A- 0 227 050
- EP-A- 0 320 320
- EP-A- 0 396 335
- EP-A- 0 531 964
- DE-A- 2 834 226
- GB-A- 2 057 878
- US-A- 2 052 376
- US-A- 4 824 677

## Description

La présente invention concerne un comprimé vétérinaire permettant de faciliter l'absorption de substances médicamenteuses par des animaux domestiques et en particulier par des chats.

Le nombre des animaux domestiques tels que chiens et chats est en augmentation constante. Parallèlement à cette augmentation, on assiste à une évolution des soins apportés à ces animaux.

Il faut constater qu'à ce jour, le suivi des traitements administrés n'est pas toujours bien assuré, en raison de la difficulté à faire absorber les médications à l'animal.

On s'est rendu compte, conformément à l'invention, que chez le chat, une des raisons de cette difficulté est liée à la forme ronde des comprimés conventionnels : en effet, l'animal à qui l'on propose de tels comprimés a tendance à les faire rouler, ce qui déclenche, chez lui, un réflexe de jeu dont la conséquence est un refus de l'administration.

On a ainsi été amené à vérifier que, de manière surprenante, la forme des comprimés influe dans une large mesure sur leur caractère appétent pour les chats.

L'objet de l'invention est d'utiliser cette constatation pour proposer un comprimé vétérinaire, plus spécialement destiné aux chats, qui soit susceptible d'être absorbé spontanément par l'animal.

Selon l'invention, un tel comprimé est caractérisé en ce qu'il présente une forme oblongue totalement dépourvue de symétrie de rotation.

Selon une caractéristique préférentielle de l'invention, ce comprimé comporte deux faces principales identiques essentiellement ovales séparées par une surface cylindrique latérale dont la génératrice est essentiellement perpendiculaire aux faces principales.

On a pu vérifier qu'un tel comprimé - dont, pour des raisons de facilité de fabrication, les faces principales sont souvent légèrement bombées vers l'extérieur - est facilement accepté par l'animal de par son caractère non rotatif, mais également grâce à une préhension plus aisée elle aussi de nature à augmenter l'appétence.

On a pu constater, selon une autre caractéristique particulièrement avantageuse de l'invention, que celle-ci peut encore être améliorée en prévoyant un resserrement à la partie médiane du comprimé.

La présence d'un tel resserrement s'avère en effet de nature à faciliter, dans une large mesure, la préhension du comprimé par le chat.

Il est, par ailleurs, habituel de proposer à la vente, que ce soit dans le domaine médical ou dans le domaine vétérinaire, des comprimés sécables permettant de respecter plus spécialement le schéma posologique par kg de poids corporel du patient ou de l'animal à traiter : il est en effet souvent nécessaire de ne distribuer que des moitiés de comprimé pour adapter la posologie.

Or, on s'est rendu compte, selon une autre caractéristique de l'invention, que le fait d'équiper l'une des faces principales du comprimé à sa partie médiane, d'une entaille faisant office de barre de sécabilité, n'enlève rien à la facilité de préhension du comprimé par le chat.

Il est donc particulièrement avantageux de prévoir de telles entailles.

Les dimensions du comprimé vétérinaire susmentionné, bien que non limitatives de l'invention, doivent, bien entendu, être adaptées à la taille moyenne des chats auxquels ceux-ci sont plus spécialement destinés.

Pour cette raison, les faces principales du comprimé ont, en moyenne, une longueur de l'ordre de 6 à 15 mm pour une largeur maximale de l'ordre de 4 à 6 mm ; la largeur au niveau du resserrement est, quant à elle, habituellement de l'ordre de 3,5 à 5,5 mm, ce qui signifie qu'il y en moyenne, environ 0,5 mm d'écart entre la largeur maximale et la largeur minimale au niveau du resserrement.

Des essais effectués à partir de comprimés ayant 11 mm de longueur, 5 mm de largeur maximale et 4,5 mm de largeur au niveau du resserrement, ont permis de mettre en évidence des caractéristiques d'appétence satisfaisantes chez la quasi totalité des chats.

L'épaisseur du comprimé peut varier dans une mesure relativement large en fonction de la nature du traitement préventif ou curatif auquel il est destiné et du type de principe actif qu'il doit contenir ; on opte néanmoins habituellement pour des dimensions permettant d'obtenir, pour chaque comprimé, un poids moyen de l'ordre de 120 à 200 mg.

Bien entendu, le comprimé vétérinaire conforme à l'invention peut renfermer différents types de principes actifs administrables par voie orale et choisis dans toutes les classes thérapeutiques et diététiques existantes et à venir, parmi lesquels on peut mentionner les exemples suivants :
Exemples de classes thérapeutiques :
- Anti-infectieux (antibiotiques, sulfamides ...)
- Anti-parasitaires internes,
- Anti-inflammatoires et anti-histaminiques,
- Vaccins oraux,
- Hormones,
- Substances de thérapeutique digestive, telles les pansements et sédatifs gastro intestinaux, les flores de remplacement, les anti-diarrhéiques, les hépato-protecteurs, les anti-spasmodiques, les laxatifs, les antiseptiques intestinaux, les réhydratants oraux ...
- Substances de thérapeutiques cardio-vasculaire telles les analeptiques cardiaques et cardio-vasculaires, les hémastotiques, les vasoconstricteurs et dilatateurs,...
- Substances de thérapeutique respiratoire telles les analeptiques respiratoires, les antitussifs, les bronchodilatateurs, les bronchosécrétolytiques, les antiseptiques respiratoires,
- Substances agissant sur le système nerveux analgésiques, sédatifs et tranquillisants, anti-épileptiques, anesthésiques, orexigènes et anorexigènes ...
- Substances de thérapeutique immunitaires telles les immunodépresseurs, les immunostimulants, les immunoglobines de remplacement ...
- Diurétiques,
- Substances anticancéreuses telles les antimitotiques.
- Les macro et oligo-éléments,
- Les vitamines,
- Les acides aminés et les protéines,
- Les acides gras,
- Les glucides,
- Les extraits de plantes ou d'organes d'animaux.

Indépendamment de ce qui précède, il est connu que de nombreuses substances de traitement ou de prévention des maladies et des déséquilibres organiques exercent un effet répulsif pour l'odorat des chats qui refusent de les absorber. Pour remédier à cet inconvénient, et de façon connue en elle-même, on peut prévoir, conformément à l'invention, d'associer le ou les principes actifs présents dans le comprimé à une matrice appétente telle que par exemple un mélange de poudre de foie et de levure de bière qui s'est avéré particulièrement prisé par les chats ou encore un mélange de différentes substances protéinées animales et/ou végétales ; on a notamment pu constater que l'usage de farine de poisson peut augmenter l'appétence de certains principes actifs.

Les principes actifs peuvent être simplement incorporés dans une telle matrice ou façonnés en un noyau lui-même englobé dans celle-ci d'une manière notamment connue par le document EP-0 320 320 B1.

A titre d'exemple non limitatif, le comprimé conforme à l'invention peut, avantageusement, comporter à titre de principe actif de l'amoxicilline, notamment sous forme trihydratée à une dose de 40 mg pour 175 mg de comprimé.

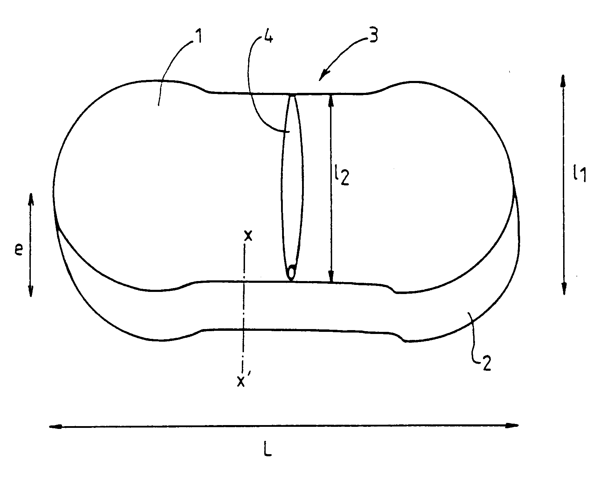

Les caractéristiques du comprimé vétérinaire qui fait l'objet de l'invention seront décrites plus en détail en se référant au dessin annexé qui est une représentation schématique d'un tel comprimé à échelle très fortement agrandie.

Selon la figure, le comprimé, de forme générale oblongue, comporte deux faces principales 1 identiques dont une seule est visible, qui sont séparées par une surface cylindrique latérale 2 dont la génératrice (x-x') est essentiellement perpendiculaire aux faces principales 1. Ces dernières, de forme essentiellement ovale, sont légèrement bombées vers l'extérieur, et comportent, à leur partie médiane un resserrement 3 facilitant la préhension du comprimé par les chats.

Les dimensions de ce comprimé, adaptées à la morphologie des chats, peuvent avantageusement être les suivantes :

| | |
|---|---|
| - longueur L : | 11,0 mm |
| - largeur maximale l₁ : | 5,0 mm |
| - largeur l₂ au niveau du resserrement 3 : | 4,5 mm. |

Ces dimensions ne doivent, bien entendu, pas être considérées comme étant limitatives de l'invention.

L'épaisseur e de ce comprimé peut, quant à elle, varier notablement en fonction du type de traitement auquel il est destiné.

Selon la figure, le comprimé comporte, à la partie médiane de l'une des faces principales 1, une entaille 4 faisant office de barre de sécabilité.

On a pu vérifier que le fait de diviser le comprimé en deux moitiés n'enlève rien à la facilité de préhension pour le chat.

## Revendications

1. Utilisation d'une forme oblongue totalement dépourvue de symétrie de rotation pour la réalisation d'un comprimé vétérinaire destiné à permettre de faciliter l'absorption de substances médicamenteuses par des animaux domestiques et en particulier par des chats.

2. Utilisation selon la revendication 1, caractérisée en ce que le comprimé comporte deux faces principales identiques (1) ovales séparées par une surface cylindrique latérale (2) dont la génératrice (x-x') est perpendiculaire aux faces principales (1).

3. Utilisation selon la revendication 2, caractérisée en ce que les faces principales (1) sont bombées vers l'extérieur.

4. Utilisation selon l'une quelconque des revendications 2 et 3, caractérisée en ce que le comprimé comporte un resserrement (3) à sa partie médiane.

5. Utilisation selon l'une quelconque des revendications 2 à 4, caractérisée en ce que, au moins l'une des faces principales (1), comporte à sa partie médiane une entaille (4) faisant office de barre de sécabilité du comprimé.

6. Utilisation selon l'une quelconque des revendications 2 à 5, caractérisée en ce que les faces principales (1) ont une longueur (L) de 6 à 15 mm pour une largeur maximale (l₁) de 4 à 6 mm.

7. Utilisation selon les revendications 4 et 6, caractérisée en ce que les faces principales (1) ont une largeur (l₂) de 3,5 à 5,5 mm au niveau du resserrement (3).

8. Utilisation selon l'une quelconque des revendications 1 à 7, caractérisée en ce que le comprimé présente un poids moyen de 120 à 200 mg.

9. Utilisation selon l'une quelconque des revendications 1 à 8, caractérisée en ce que le comprimé comporte un ou plusieurs principes actifs incorporés ou englobés dans une matrice appétente renfermant notamment un mélange de poudre de foie et de levure de bière ou encore un mélange de différentes substances protéinées d'origine animale et/ou végétale.

10. Utilisation selon l'une quelconque des revendications 1 à 9, caractérisée en ce que le comprimé comporte en tant que principe actif de l'amoxicilline, notamment sous forme trihydratée.

## Claims

1. Use of an oblong shape totally devoid of rotational symmetry for forming a veterinary tablet for facilitating the absorption of medicinal substances by domestic animals and especially cats.

2. Use according to Claim 1, characterised in that the tablet comprises two identical oval principal faces (1) which are separated by a lateral cylindrical surface (2), the generatrix (x-x') of which is perpendicular to the principal faces (1).

3. Use according to claim 2, characterised in that the principal faces (1) bulge outwards.

4. Use according to either Claim 2 or Claim 3, characterised in that the tablet comprises a narrowed region (3) at its central portion.

5. Use according to any one of Claims 2 to 4, characterised in that at least one of the principal faces (1) comprises at its central portion a notch (4) acting as a dividing groove for the tablet.

6. Use according to any one of Claims 2 to 5, characterised in that the principal faces (1) have a length (L) of from 6 to 15 mm and a maximum width (l₁) of from 4 to 6 mm.

7. Use according to Claims 4 and 6, characterised in that the principal faces (1) have a width (l₂) of from 3.5 to 5.5 mm at the narrowed region (3).

8. Use according to any one of Claims 1 to 7, characterised in that the tablet has an average weight of from 120 to 200 mg.

9. Use according to any one of Claims 1 to 8, characterised in that the tablet comprises one or more active ingredients incorporated or included in an appetence-inducing matrix containing, especially, a mixture of liver powder and brewers' yeast or a mixture of various protein substances of animal and/or vegetable origin.

10. Use according to any one of Claims 1 to 9, characterised in that the tablet comprises amoxicillin, especially in trihydrated form, as the active ingredient.

## Patentansprüche

1. Verwendung einer länglichen Form, der man jegliche Rotationssymmetrie genommen hat, zum Realisieren einer Tierarzneitablette, die dazu bestimmt ist, die Absorption von medikamentösen Substanzen durch Haustiere und insbesondere durch Katzen zu erleichtern.

2. Verwendung nach Anspruch 1, dadurch gekennzeichnet, daß die Tablette zwei identische ovale Hauptflächen (1) hat, die durch eine zylindrische Seitenfläche (2) getrennt sind, deren Erzeugende (x-x') senkrecht zu den Hauptflächen (1) liegt.

3. Verwendung nach Anspruch 2, dadurch gekennzeichnet, daß die Hauptflächen (1) nach außen gewölbt sind.

4. Verwendung nach einem der Ansprüche 2 und 3, dadurch gekennzeichnet, daß die Tablette in ihrem mittleren Bereich eine Verengung (3) hat.

5. Verwendung nach einem der Ansprüche 2 bis 4, dadurch gekennzeichnet, daß zumindest eine der Hauptflächen (1) in ihrem mittleren Bereich eine Kerbe (4) hat, die als Linie zum Teilen der Tablette dient.

6. Verwendung nach einem der Ansprüche 2 bis 5, dadurch gekennzeichnet, daß die Hauptflächen (1) eine Länge (L) von 6 bis 15 mm bei einer maximalen Breite (l₁) von 4 bis 6 mm haben.

7. Verwendung nach den Ansprüchen 4 und 6, dadurch gekennzeichnet, daß die Hauptflächen (1) in Höhe der Verengung (3) eine Breite (l₂) von 3,5 bis 5,5 mm haben.

8. Verwendung nach einem der Ansprüche 1 bis 7, dadurch gekennzeichnet, daß die Tablette ein durchschnittliches Gewicht von 120 bis 200 mg hat.

9. Verwendung nach einem der Ansprüche 1 bis 8, dadurch gekennzeichnet, daß die Tablette einen oder mehrere Wirkstoffe enthält, die in eine appetitanregende Grundmasse integriert oder eingeschlossen sind, die insbesondere ein Gemisch aus pulverförmiger Leber und Bierhefe oder noch ein Gemisch aus verschiedenen proteinhaltigen Substanzen tierischen und/oder pflanzlichen Ursprungs enthält.

10. Verwendung nach einem der Ansprüche 1 bis 9, dadurch gekennzeichnet, daß die Tablette als Wirkstoff Amoxicillin enthält, insbesondere in trihydrierter Form.
